# EUROPEAN PATENT SPECIFICATION

(11) **EP 3 549 679 B1**
(45) Date of publication and mention of the grant of the patent: **16.07.2025**
(21) Application number: 19174997.7
(22) Date of filing: 07.05.2014
(51) Int. Cl.: B05C 5/02, A61M 25/10

(54) **APPARATUS AND METHODS FOR COATING MEDICAL DEVICES**
VORRICHTUNG UND VERFAHREN ZUR BESCHICHTUNG MEDIZINISCHER VORRICHTUNGEN
APPAREIL ET PROCÉDÉS DE REVÊTEMENT DE DISPOSITIFS MÉDICAUX

(30) Priority: 07.05.2013 US 201361820223 P; 31.05.2013 US 201361829375 P; 31.05.2013 US 201313906599; 09.09.2013 US 201361875524 P
(43) Date of publication of application: 09.10.2019
(62) Divisional of application: 14730319.2
(73) Proprietor: SurModics, Inc., Eden Prairie, MN 55344-3523 (US)
(72) Inventor: Chappa, Ralph A., Ham Lake, MN Minnesota 55304 (US); Bach, Andrew G., Edina, MN Minnesota 55436 (US); MacGregor, Mark, St. Paul, MN Minnesota 55107 (US)
(74) Representative: Potter Clarkson

(56) References cited:
- WO-A2-2010/024898
- JP-A- 2005 059 225
- US-A1- 2004 211 362
- US-A1- 2011 281 019

## Description

### Field of the Invention

The present invention relates to apparatus and methods for coating medical devices.

### Background of the Invention

Functional improvements to implantable or insertable medical devices can be achieved by coating the surface of the device. For example, a coating formed on the surface of the device can provide improved lubricity, improved biocompatibility, or drug delivery properties to the surface. In turn, this can improve movement of the device in the body, extend the functional life of the device, or treat a medical condition near the site of implantation. However, various challenges exist for the design and use of coating apparatus designed to provide coatings to medical devices.

Traditional coating methods, such as dip coating, are often undesirable as they may result in flawed coatings that could compromise the function of the device or present problems during use. These methods can also result in coating inaccuracies, which can be manifested in variable amounts of the coated material being deposited on the surface of the device. When a drug is included in the coating material, it is often necessary to deliver precise amounts of the agent to the surface of the device to ensure that a subject receiving the coated device receives a proper dose of the agent. It has been difficult to achieve a great degree of accuracy using traditional coating methods and machines.

One type of insertable medical device is a balloon catheter. Balloon catheter constructions are well known in the art and are described in various documents, for example, U.S. Pat. Nos. 4,195,637, 5,041,089, 5,087,246, 5,318,587, 5,382,234, 5,571,089, 5,776,101, 5,807,331, 5,882,336, 6,394,995, 6,517,515, 6,623,504, 6,896,842, and 7,163,523. Balloon catheters generally include four portions, the balloon, catheter shaft, guide wire, and manifold. A balloon catheter generally includes an elongated catheter shaft with an inflatable balloon attached to a distal section of the catheter shaft. At a proximal end of the catheter shaft, there is typically a manifold. At the manifold end, placement of the catheter can be facilitated using a guide wire. Guide wires are small and maneuverable when inserted into an artery. Once the guide wire is moved to the target location, the catheter with balloon portion is then fed over the guide wire until the balloon reaches the target location in the vessel. The balloon is typically inserted into the arterial lumen of a patient and advanced through the lumen in an unexpanded state. The balloon is then inflated when the catheter reaches target site resulting in application of mechanical force sufficient to cause vessel dilation. The balloon is typically inflated using a fluid, which is injected through an inflation port. The manifold can control the fluid introduction within shaft for expansion of the balloon. The mechanics of fluid transfer and introduction within balloons vary according to the specific design of the catheter, and are well known in the art.

US2011/281019A1 describes a system and method for coating an expandable member of a medical device which comprises providing a dispenser in fluid communication with a fluid source with the dispenser having at least one outlet to dispense fluid of the fluid source therefrom. The outlet(s) of the dispenser is positioned proximate a surface of an expandable member, with relative movement between the outlet(s) and the surface of the expandable member established along a coating path, and fluid is dispensed from the dispenser to form a substantially continuous bead of fluid between the at least one outlet and the surface of the expandable member along the coating path, and simultaneously drying the fluid while dispensing the fluid from the dispenser to control flow of fluid on the surface of the expandable member. The fluid source can include a variety of therapeutic agents.

WO2010/024898A2 describes a method and apparatus for coating a medical device. In one embodiment, the method for preparing a substantially uniform coated medical device includes (1) preparing a coating solution comprising a solvent, a therapeutic agent, and an additive; (2) loading a metering dispenser with the coating solution; (3) rotating the medical device about the longitudinal axis of the device and/or moving the medical device along the longitudinal or transverse axis of the device; (4) dispensing the coating solution from the metering dispenser onto a surface of the medical device and flowing the coating solution on the surface of the medical device while the medical device is rotating and/or linearly moving; and (5) evaporating the solvent, forming a substantially uniform coating layer on the medical device.

### Summary

The present invention comprises a coating apparatus and a method of coating a medical device as defined by the claims. Embodiments that do not fall within the scope of the claims are to be interpreted as examples useful for understanding the invention.

Embodiments include apparatus and methods for coating drug coated medical devices. An embodiment which is disclosed but not claimed includes a coating apparatus including a coating application unit comprising a movement restriction structure; a fluid applicator; and an air nozzle. The apparatus can further include a rotation mechanism and a axial motion mechanism, the axial motion mechanism configured to cause movement of at least one of the coating application unit and the rotation mechanism with respect to one another.

Another embodiment which is disclosed but not claimed includes a coating apparatus including a coating application unit comprising a fluid applicator; a fluid distribution bar; an air nozzle; and a rotation mechanism. The coating apparatus can further include an axial motion mechanism, the axial motion mechanism configured to cause movement of the coating application unit with respect to the rotator.

Another embodiment which is disclosed but not claimed includes a method of coating including rotating a balloon catheter with a rotation mechanism, the balloon catheter comprising a balloon, contacting the balloon with a movement restriction structure defining a channel; applying a coating solution onto the surface of the balloon with a fluid applicator, contacting the surface of the balloon with a fluid distribution bar, blowing a stream of a gas onto the surface of the balloon, wherein the channel limits lateral movement of the balloon.

Another embodiment includes a coating apparatus. The coating apparatus includes a coating application unit. The coating application unit includes a movement restriction structure, a fluid applicator having a lengthwise axis and a width. The fluid applicator includes a tip. The tip includes a face across the width of the fluid applicator. The face is oriented at an angle of from about 105 to about 165 degrees with respect to the lengthwise axis of the fluid applicator. The fluid applicator is configured to rotate around its lengthwise axis so as to change the orientation of the face with respect to the device being coated. The coating apparatus further includes a rotation mechanism and an axial motion mechanism. The axial motion mechanism is configured to cause movement of at least one of the coating application unit and the rotation mechanism with respect to one another. The fluid applicator is configured to rotate around its lengthwise axis by an amount from about 10 degrees to about 170 degrees.

Another embodiment includes a method of coating a medical device. The method includes engaging a medical device to be coated into a movement restriction structure and rotating a medical device to be coated with a rotation mechanism. The method further includes contacting the surface of the medical device with a fluid applicator having a lengthwise axis and a width. The fluid applicator includes a tip. The tip includes a face across the width of the fluid applicator. The face is oriented at an angle of from about 105 to about 165 degrees with respect to the lengthwise axis of the fluid applicator. The method includes applying a coating solution onto the surface of the balloon with the fluid applicator. The method further includes rotating the fluid applicator about its lengthwise axis by an amount from about 10 to about 170 degrees.

Another embodiment which is disclosed but not claimed includes a coated medical device including a shaft, an expandable portion having a surface, and a coating disposed on the expandable portion. The coating can include a continuous coverage segment and a discontinuous coverage segment.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope of the present invention is defined by the appended claims and their legal equivalents.

### Brief Description of the Figures

The invention may be more completely understood in connection with the following drawings, in which:
FIG. 1 is a schematic side view of a coating apparatus in accordance with various embodiments herein.
FIG. 2 is a schematic view of a coating application unit in accordance with various embodiments herein.
FIG. 3 is a schematic view of a movement restriction structure in accordance with various embodiments herein.
FIG. 4 is a schematic view of a movement restriction structure in accordance with various embodiments herein.
FIG. 5 is a schematic view of a movement restriction structure in accordance with various embodiments herein.
FIG. 6 is a schematic end view of a fluid distribution bar in conjunction with the balloon of a balloon catheter.
FIG. 7 is a schematic end view of a fluid applicator in conjunction with the balloon of a balloon catheter.
FIG. 8 is a schematic end view of an air nozzle in conjunction with the balloon of a balloon catheter.
FIG. 9 is a schematic view of a coating application unit in accordance with various embodiments herein.
FIG. 10 is a schematic view of a coating application unit in accordance with various embodiments herein.
FIG. 11 is a schematic top view of a movement restriction structure in accordance with various embodiments herein.
FIG. 12 is a schematic end view of a movement restriction structure in accordance with various embodiments herein.
FIG. 13 is a schematic front view of a movement restriction structure in accordance with various embodiments herein.
FIG. 14 is a schematic front view of a movement restriction structure in accordance with various embodiments herein.
FIG. 15 is a schematic end view of a movement restriction structure in accordance with various embodiments herein.
FIG. 16 is a schematic end view of a fluid applicator in accordance with various embodiments herein.
FIG. 17 is a schematic perspective view of a coating apparatus in accordance with various embodiments herein.
FIG. 18 is a schematic perspective view of a fluid applicator interfacing with balloon catheter as held by a movement restriction structure.
FIG. 19 is a schematic perspective view of a fluid applicator interfacing with balloon catheter as held by a movement restriction structure.
FIG. 20 is a schematic perspective view of elements of a coating apparatus in accordance with various embodiments herein.
FIG. 21 is a schematic view through the middle of a fluid applicator in accordance with various embodiments herein.
FIG. 22 is a schematic cross-sectional view of a fluid applicator interfacing with balloon catheter as held by a movement restriction structure.
FIG. 22A is a schematic cross-sectional view of a fluid applicator interfacing with balloon catheter as held by a movement restriction structure in accordance with various embodiments.
FIG. 23 is a schematic cross-sectional view of a fluid applicator interfacing with a balloon catheter in accordance with various embodiments
FIG. 24 is a schematic top view of a fluid applicator interfacing with a balloon catheter in accordance with various embodiments.
FIG. 25 is a schematic top view of a fluid applicator interfacing with a balloon catheter in accordance with various embodiments.
FIG. 26 is a schematic top view of a fluid applicator interfacing with a balloon catheter in accordance with various embodiments.
FIG. 27 is a schematic top view of a fluid applicator interfacing with a balloon catheter in accordance with various embodiments.
FIG. 28 is a schematic top view of a fluid applicator interfacing with a balloon catheter in accordance with various embodiments.
FIG. 29 is a schematic cross-sectional view of a fluid applicator.
FIG. 30 is a schematic view of a fluid applicator interfacing with a surface of a device to be coated.
FIG. 31 is a schematic top view of a fluid applicator interfacing with a surface of a device to be coated.
FIG. 32 is a schematic top view of a fluid applicator interfacing with a surface of a device to be coated.
FIG. 33 is a schematic top view of a fluid applicator interfacing with a surface of a device to be coated.
FIG. 34 is a schematic view of a portion of a medical device in accordance with various embodiments herein.
FIG. 35 is a schematic view of a medical device in accordance with various embodiments herein.

While the invention is susceptible to various modifications and alternative forms, specifics thereof have been shown by way of example and drawings, and will be described in detail. It should be understood, however, that the invention is not limited to the particular embodiments described. On the contrary, the intention is to cover modifications, equivalents, and alternatives falling within the scope of the claims.

### Detailed Description

The embodiments described herein are not intended to be exhaustive or to limit the invention to the precise forms disclosed in the following detailed description. Rather, the embodiments are chosen and described so that others skilled in the art can appreciate and understand the principles and practices of the present invention.

The publications and patents disclosed herein are provided solely for their disclosure. Nothing herein is to be construed as an admission that the inventors are not entitled to antedate any publication and/or patent, including any publication and/or patent cited herein.

Embodiments herein can be used to apply visually uniform coatings, such as coatings including active agents, onto medical devices, such as onto the balloons of drug coated or drug eluting balloon catheters, that have substantially uniform active agent concentrations along the length of the medical device. For example, in some embodiments, coatings can be formed with apparatus and methods wherein each section of the device that has been coated contains an amount of the active agent that is within ten percent of the average amount of active agent across all sections coated.

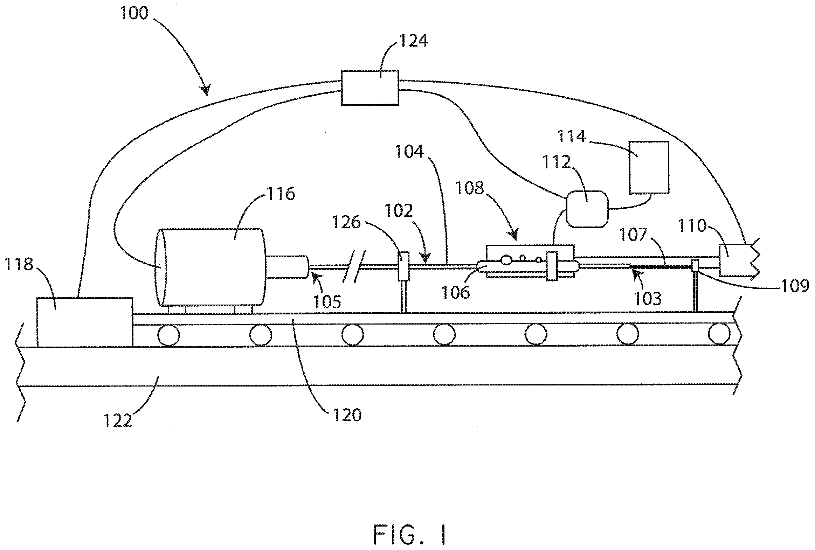

Referring now to FIG. 1, a schematic side view is shown of a coating apparatus 100 in accordance with various embodiments herein. The coating apparatus 100 is shown in conjunction with a drug coated balloon catheter 102. The drug coated balloon catheter 102 can include a catheter shaft 104 and a balloon 106. The balloon 106 can assume a deflated configuration and an inflated configuration. The drug coated balloon catheter 102 can include a distal end 103 and a proximal end 105. The drug coated balloon catheter 102 can include a proximal end manifold (not shown). The coating apparatus 100 can include a coating application unit 108. The coating apparatus 100 can further include, in some embodiments, an axial motion mechanism 110 (axial with respect to the axis of rotation of the balloon catheter and thus parallel to the lengthwise axis of the balloon catheter) that can function to move one or more components of the coating application unit 108. In some embodiments, axial motion can be substantially horizontal. In other embodiments, axial motion can be substantially vertical. In some embodiments, axial motion can be somewhere in between horizontal and vertical, depending on the orientation of the lengthwise axis of the balloon catheter. However, it will be appreciated that in other embodiments, the coating application unit 108 can remain stationary.

Coating of the balloon 106 to make it drug coated can occur starting at the proximal end of the balloon and proceeding to the distal end. However, in other embodiments, coating of the drug coated balloon 106 can occur starting at the distal end of the balloon and proceeding to the proximal end. In many embodiments, coating can take place with a single pass of the coating application unit 108 with respect to the balloon. However, in other embodiments, multiple passes of the coating. application unit with respect to the balloon can be made.

The coating apparatus 100 can further include a fluid pump 112. The fluid pump 112 can be, for example, a syringe pump. The fluid pump 112 can be in fluid communication with components of the coating application unit 108 (such as the fluid applicator) and with a fluid reservoir 114. The fluid pump 112 can operate to pump a coating solution at a rate sufficient to apply about 0.5 µl to about 10 µl of the coating solution per millimeter of length of the balloon or other device to be coated. The coating apparatus 100 can further include a rotation mechanism 116 (or rotating balloon catheter fixture). The rotation mechanism 116 can be directly or indirectly coupled to the drug coated balloon catheter in order to rotate the drug coated balloon catheter 102 around its lengthwise (major) axis (about the central lumen of the catheter). In some embodiments, the drug coated balloon catheter can be rotated at a speed of between 100 and 400 rotations per minute. In some embodiments, the drug coated balloon catheter can be rotated at a speed of between 200 and 300 rotations per minute.

In some embodiments, a guide wire 107, passing through the central lumen of the catheter, can extend from the distal tip of the catheter and be inserted into a distal tip support ring 109 or guide. In this manner, the guide wire 107 can be used to support the distal tip of the balloon catheter to be coated while allowing the balloon catheter to rotate freely.

The coating apparatus 100 can further include, in some embodiments, an axial motion mechanism 118 which can be configured to move the drug coated balloon catheter 102 in the direction of its lengthwise major axis. In some embodiments, axial motion can be substantially horizontal. In other embodiments, axial motion can be substantially vertical. In some embodiments, axial motion can be somewhere in between horizontal and vertical, depending on the orientation of the lengthwise axis of the balloon catheter. In some embodiments, the axial motion mechanism 118 can be a linear actuator. In some embodiments, the axial motion mechanism 118 can include an electric motor. The coating apparatus 100 can further include a frame member 120 (in some embodiments this can also be referred to as an axial motion support rail). The frame member 120 can support other components of the coating apparatus 100 such as one or more guides 126. The frame member 120 can itself be support by a platform 122. The coating apparatus 100 can further include a controller 124 that can serve to control operation of the coating apparatus 100 including, specifically, fluid pump 112, axial motion mechanism 110, rotation mechanism 116, and axial motion mechanism 118.

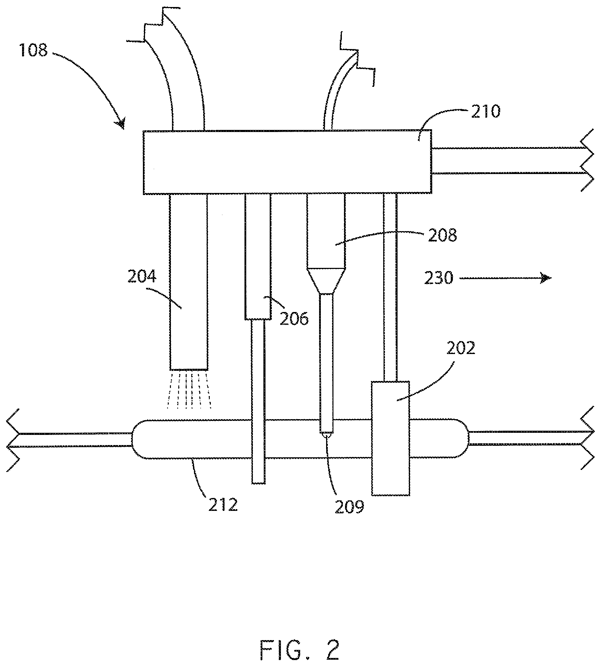

Referring now to FIG. 2, a schematic view of a coating application unit 108 in accordance with various embodiments herein is shown. The coating application unit 108 can include a movement restriction structure 202 (or wobble control structure), an air nozzle 204, a fluid distribution bar 206, and a fluid applicator 208. The movement restriction structure 202 can serve to limit the lateral motion (e.g., movement in a direction perpendicular to the lengthwise axis of the catheter) of the balloon during a coating operation.

The fluid applicator 208 can serve to apply a coating solution 209 to the surface of the balloon 212 on the drug coated balloon catheter. In some embodiments, the fluid applicator 208 is less than or equal to about 1 cm away from the movement restriction structure 202. In some embodiments, the air nozzle 204 is less than or equal to about 2 cm away from the fluid applicator 208. The air nozzle 204 can provide a stream of a gas in order to assist in drying the coating solution after it has been applied to the balloon or other medical device.

The fluid distribution bar 206 can serve to promote distribution of the applied coating solution. For example, the fluid distribution bar 206 can serve to prevent pooling of the applied coating solution. In some embodiments, the fluid distribution bar 206 can be at least about 0.5 mm away from the fluid applicator and less than 2 cm away. In some embodiments, the fluid distribution bar 206 can be at least about 0.2 cm away from the fluid applicator and less than 2 cm away.

In this embodiment, the coating application unit 108 can move, relative to the balloon 212 in the direction of arrow 230. As such, during a coating operation, the movement restriction structure 202 can pass over the balloon first, followed by the fluid applicator 208, followed by the fluid distribution bar 206, with the air nozzle last. It should be emphasized, however, that this movement is relative in the sense that in some embodiments the coating application unit 108 is moving and the balloon 212 is rotating but otherwise stationary, in some embodiments the balloon 212 is rotating and moving in the direction of its lengthwise axis and the coating application unit 108 is stationary, in still other embodiments both the coating application unit 108 and the balloon 212 are moving. The speed of movement of the balloon 212 relative to the coating application unit 108 can vary depending on the amount of coating solution to be applied. In some embodiments the speed can be from about 0.02 centimeters per second to about 0.2 centimeters per second.

It will be appreciated that based on the rotation of the drug coated balloon catheter and the movement of the balloon relative to the coating application unit that the path of the deposition of the coating onto the balloon follows a roughly helical path. It will be appreciated that the combination of the rotation speed of the drug coated balloon catheter and the speed of the movement of the balloon relative to the coating application unit can influence the amount of coating solution that is deposited at any given point and the nature of the helical path. For example, the coating material can be deposited in helical layers that partially overlap one another at their edges, helical layers wherein the edge of one turn substantially meets the edge of a previous turn, and helical layers wherein there are gaps in between subsequent helical turns. In some embodiments, these helical patterns can be configured so as to maximize release of the active agent. For example, in some embodiments, the apparatus can be used to coat device so as to produce helical ridges of the coating material on the balloon surface.

In some embodiments, the coating application unit 108 can optionally include a manifold block 210. The manifold block 210 can facilitate support of, and in some embodiments movement of, the components of the coating application unit 108. In some embodiments, the components of the coating application unit can move together as a unit during a coating operation. However, in other embodiments the components of the coating application unit are substantially separate from one another and can move independently. In some embodiments, the components of the coating application unit are all substantially stationary during a coating operation.

While the components of the coating application unit 108 are shown in FIG. 2 as being within a particular plane and disposed at approximately the same angle with respect to the balloon 212 being coated, it will be appreciated that this is not the case with all embodiments herein. In some embodiments, the components of the coating application unit 108 lie in different planes with respect to the balloon 212 and/or the components of the coating application unit 108 are disposed at different angles (both with respect to the lengthwise axis of the balloon and radially) with respect to the balloon.

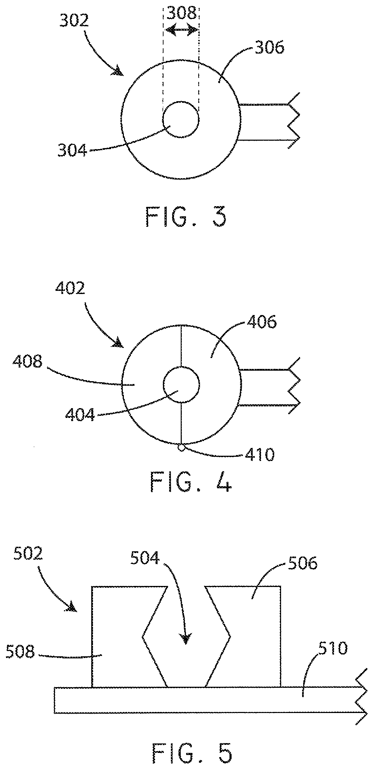

Referring now to FIG. 3, a schematic end view is shown of a movement restriction structure 302 in accordance with various embodiments herein. The structure 302 can include a body member 306 defining a channel 304 or aperture. The body member 306 can be formed of various materials such as polymers, metals, ceramics, and the like. In a particular embodiment, the body member 306 is formed of polytetrafluoroethylene (PTFE). The channel 304 can have a diameter 308 that is sufficiently large so as to accommodate the balloon of a drug coated balloon catheter in an expanded state. In the example of FIG. 3, the channel 304 is shown as being bounded in a radially continuous manner by the body member 306 (e.g., it is completely surrounded on all sides by the body member 306). However, it will be appreciated that in some embodiments the channel 304 is not bounded in a radially continuous manner by the body member 306.

In some embodiments the movement restriction structure can include multiple pieces that together define a channel or aperture. Referring now to FIG. 4, a movement restriction structure 402 is shown including a body member that includes a first piece 406 and a second piece 408 that together define a channel 404 or aperture. The first piece 406 and second piece 408 are joined together by a hinge 410 in this embodiment, however it will be appreciated that there are many ways known to those of skill in the art by which to hold two structure pieces in association with one another.

It will be appreciated that body members of movement restriction structures can take on many different shapes. In addition, the shape of the channel defined by the body member(s) can take on many different shapes. Referring now to FIG. 5, a movement restriction structure 502 is shown including a first side piece 506 and a second side piece 508 that together define a channel 504 or aperture. In this case, the first side piece 506 and the second side piece 508 are supported by a frame member 510. However, it will be appreciated that there are many different ways of supporting the first side piece 506 and the second side piece 508. In some embodiments, one or both of the first side piece 506 and the second side piece 508 can be spring loaded such that it is biased toward sliding inward toward the other piece. In other embodiments, one or both of the first side piece 506 and the second side piece 508 can be adjustable and then fixed in position so as to create a channel 504 of a desired size.

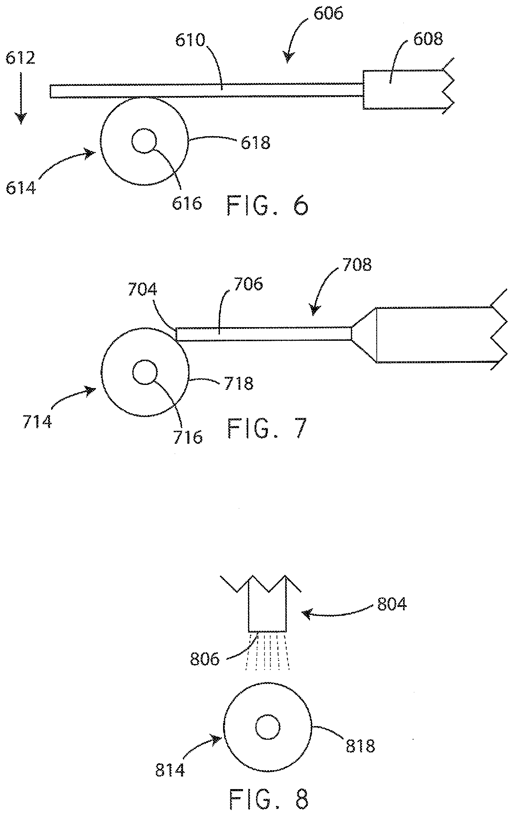

Referring now to FIG. 6 a schematic end view of a fluid distribution bar 606 in conjunction with the balloon 618 of a drug coated balloon catheter 614 is shown. In some embodiments, the fluid distribution bar 606 can include a support structure 608 and a shaft 610. In some embodiments, the support structure 608 can be omitted. The shaft 610 can be formed of various materials such as polymers, metals, ceramics, and the like. In a particular embodiment, the shaft 610 is formed of polytetrafluoroethylene (PTFE). The shaft 610 can be of various lengths and diameters and can have various cross-sectional shapes. In some embodiments, the shaft 610 is from about 2 mm to about 15 cm and is substantially circular in cross-sectional shape. In some embodiments, the shaft is about 1/16 inch (1.6 mm) in diameter. The shaft 610 is configured to rest against the balloon 618 of the balloon catheter 614.

In yet other embodiments the fluid distribution bar 606 can include multiple rods or extensions from support structure 608. Exemplary of these embodiments can include, but are not limited to, a comb-like structure or a brush.

The balloon 618 is supported by the catheter shaft 616, but generally only at the ends of the balloon 618. Because of the limited support of the balloon 618 by the catheter shaft 616, the inherent flexibility of the balloon material and manufacturing variations, the balloon 618 may not be perfectly round. As such, when it is being rotated during a coating operation there may be variations in the distance of the outer surface of the balloon 618 from the catheter shaft 616 of the balloon catheter 614. If unaccounted for, this could lead to circumstances where the fluid distribution bar 606 does not maintain contact with the surface of the balloon 618. As such, the shaft 610 of the fluid distribution bar 606 can be configured to maintain contact with the surface of the balloon 618. For example, the shaft 610 of the fluid distribution bar 606 can be positioned such that it exerts a small degree of pressure against the surface of the balloon 618 such that when an irregularity in the balloon is encountered the fluid distribution bar 606 can move slightly in order to maintain contact with the balloon surface. In some embodiments the shaft 610 of the fluid distribution bar 606 is flexible to accommodate movement to stay in contact with the balloon surface. In other embodiments, the fluid distribution bar 606 can be configured to pivot from where it is mounted in order to accommodate movement to stay in contact with the balloon surface.

While the shaft 610 of the fluid distribution bar 606 is shown in FIG. 6 as contacting the top of the balloon 618 and thus exerting a pressure downward in the direction of arrow 612, it will be appreciated that in other embodiments the surface of the balloon 618 can be contacted at other points along its surface, such as on the sides or on the bottom.

Referring now to FIG. 7, a schematic end view of a fluid applicator 708 in conjunction with the balloon 718 of a drug coated balloon catheter 714 is shown in accordance with an embodiment. The fluid applicator 708 can include a shaft 706 and an orifice 704. In some embodiments, the fluid applicator 708 can be a pipette. Fluid, such as a coating solution, can travel through the shaft 706 of the fluid applicator 708 in order to be deposited on the surface of the balloon 718 of the drug coated balloon catheter 714. The shaft 706 is configured to rest against the balloon 718 of the balloon catheter 714. The balloon 718 is supported by the catheter shaft 716, but generally only at the ends of the balloon 718. Because of the limited support of the balloon 718 by the catheter shaft 716, the inherent flexibility of the balloon material and manufacturing variations, the balloon 718 may not be perfectly round. As such, when it is being rotated during a coating operation there may be variations in the distance of the outer surface of the balloon 718 from the catheter shaft 716 of the balloon catheter 714. If unaccounted for, this could lead to circumstances where the fluid applicator 708 does not maintain contact with the surface of the balloon 718. As such, the shaft 706 of the fluid applicator 708 can be configured to maintain contact with the surface of the balloon 718. For example, the shaft 706 of the fluid applicator 708 can be positioned such that it exerts a small degree of pressure against the surface of the balloon 718 such that when an irregularity in the balloon 718 is encountered the fluid applicator 708 can move slightly in order to maintain contact with the balloon surface. In some embodiments the shaft 706 of the fluid applicator 708 is flexible to accommodate movement to stay in contact with the balloon surface. In other embodiments, the fluid applicator 708 can be configured to pivot from where it is mounted in order to accommodate movement to stay in contact with the balloon surface. In other embodiments, the fluid applicator may not be in direct contact with the balloon surface but situated closely, for example within 1 millimeter.

While the shaft 706 of the fluid applicator 708 is shown in FIG. 7 as contacting the upper right side (approximately equivalent to an area between the 1 and 2 position of a clock face) of the balloon 718, it will be appreciated that in other embodiments the surface of the balloon 718 can be contacted at other points along its surface. For example, in some embodiments, the very top of the balloon 718 can be contacted by the fluid applicator 708.

In some embodiments the fluid distribution bar 606 and the fluid applicator 708 can be configured such that the shaft 610 of the fluid distribution bar 606 contacts the surface of the balloon at approximately the same point radially along the surface of the balloon as the shaft 706 of the fluid applicator 708. In some embodiments, the fluid distribution bar 606 and the fluid applicator 708 can be configured such that the shaft 610 of the fluid distribution bar 606 contacts the surface of the balloon within at least 90 degrees radially along the surface of the balloon as the shaft 706 of the fluid applicator 708.

Referring now to FIG. 8, a schematic end view of an air nozzle 804 in conjunction with the balloon 818 of a drug coated balloon catheter 814 is shown. The air nozzle 804 can include an orifice 806. A gas such nitrogen, ambient air or another gas can be directed to flow out of the orifice 806 and towards the balloon 818 of the drug coated balloon catheter 814. In some embodiments, the gas can be heated. For example, in some embodiments the gas can be from about 50 to about 70 degrees Celsius. While the orifice 806 of the air nozzle 804 is shown in FIG. 8 as directing air to the top of the balloon 818, it will be appreciated that in other embodiments the air nozzle 804 and orifice 806 can be configured to direct air at other parts of the balloon 818 such as, but not limited to, the sides or the bottom.

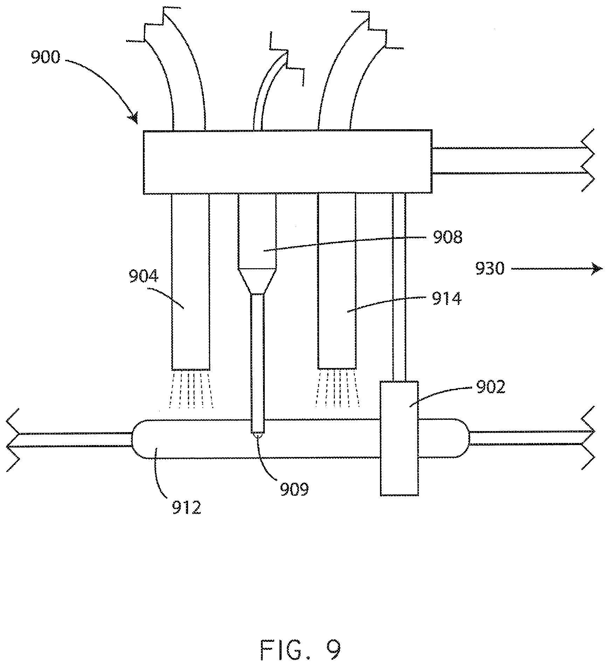

Referring now to FIG. 9, a schematic view of a coating application unit in accordance with various embodiments herein is shown. The coating application unit 900 can include a movement restriction structure 902, a first air nozzle 914, a fluid applicator 908, and a second air nozzle 904. The first air nozzle 914 is disposed on one side of the fluid applicator 908 and the second air nozzle 904 is disposed on the other side of the fluid applicator 908. In some embodiments the first air nozzle 914 can act to avoid pooling of the coating at the fluid applicator 908. In some embodiments the second air nozzle 904 can act to avoid pooling of the coating fluid at the fluid applicator 908. The fluid applicator 908 can serve to apply a coating solution 909 to the surface of the balloon on the drug coated balloon catheter. Other embodiments can include three or more air nozzles.

In this embodiment, the coating application unit 900 can move, relative to the balloon 912 in the direction of arrow 930. As such, during a coating operation, the movement restriction structure 902 can pass over the balloon first. It should be emphasized, however, that this movement is relative in the sense that in some embodiments the coating application unit 900 is moving and the balloon 912 is rotating but otherwise stationary, in some embodiments the balloon 912 is rotating and moving in the direction of its lengthwise axis and the coating application unit 900 is stationary, in still other embodiments both the coating application unit 900 and the balloon 912 are moving.

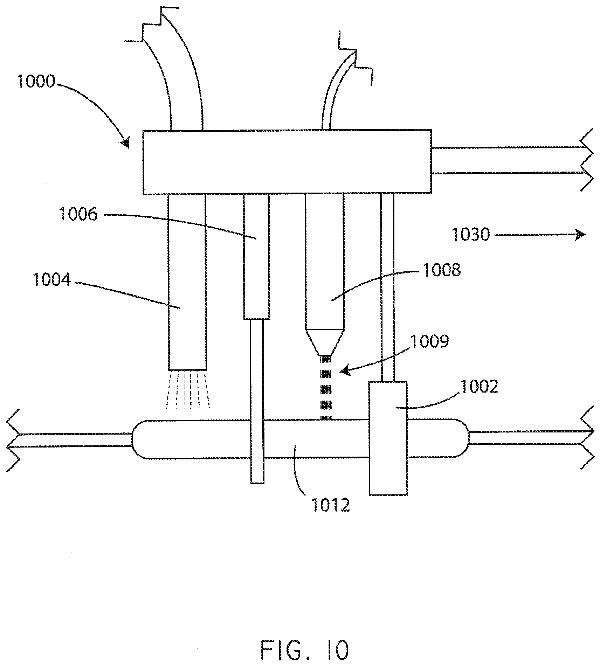

It will be appreciated that the coating solution can be applied on to the balloon in various ways including, but not limited to, spraying (including both ultrasonic spraying and conventional spraying techniques), dribbling, blade coating, contact printing, drop coating, or the like. In some embodiments, the fluid applicator can include a fluid spray nozzle. Referring now to FIG. 10, a schematic view of a coating application unit in accordance with various embodiments herein is shown. The coating application unit 1000 can include a movement restriction structure 1002, an air nozzle 1004, a fluid distribution bar 1006, and a fluid spray nozzle 1008. The fluid spray nozzle 1008 can serve to apply a coating solution 1009 to the surface of the balloon 1012 on the drug coated balloon catheter. In some embodiments there is a small gap between the fluid spray nozzle 1008 and the balloon 1012. For example, the gap can be between 1 millimeter and 10 centimeters. In some embodiments, multiple fluid applicators and/or spray nozzles can be used.

In this embodiment, the coating application unit 1000 can move, relative to the balloon 1012 in the direction of arrow 1030. As such, during a coating operation, the movement restriction structure 1002 can pass over the balloon first. It should be emphasized, however, that this movement is relative in the sense that in some embodiments the coating application unit 1000 is moving and the balloon 1012 is rotating but otherwise stationary, in some embodiments the balloon 1012 is rotating and moving in the direction of its lengthwise axis and the coating application unit 1000 is stationary, in still other embodiments both the coating application unit 1000 and the balloon 1012 are moving.

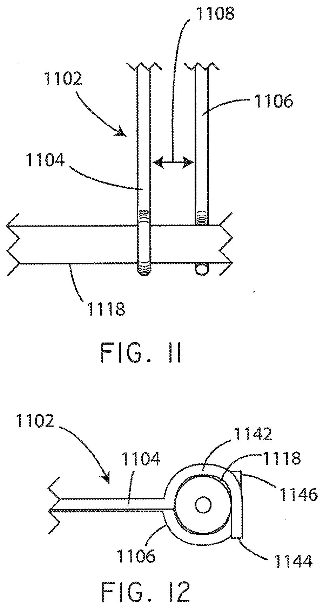

FIG. 11 is a schematic top view of a movement restriction structure in accordance with various embodiments herein. The structure 1102 can include a first body member 1104 and a second body member 1106. The first and second body members 1104, 1106 can be formed of various materials such as polymers, metals, ceramics, and the like. The first and second body members 1104, 1106 can function together to restrict movement of a balloon 1118 to be coated. The first and second body members 1104, 1106 can be separated from one another by a distance 1108 that is greater than or equal to the diameter of the balloon 1118. In some embodiments, the distance 1108 is approximately equal to the balloon 1118. In some embodiments, the distance 1108 is between about 3 millimeters and about 10 millimeters.

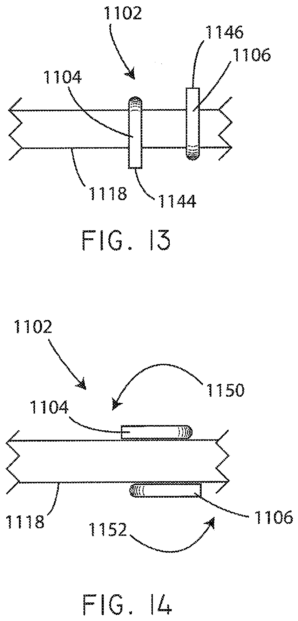

FIG. 12 is a schematic end view of the movement restriction structure 1102. The first body member 1104 can include a curved segment 1142 and an end 1144. The curved segment 1142 can define a portion of a channel which can surround at least a portion of the balloon 1118, thereby restricting its movement. In some embodiments, the second body member 1106 can be formed similarly but with a different orientation so that together the first body member 1104 and the second body member 1106 can effectively restrict movement of the balloon 1118. For example, the end 1146 of the second body member 1106 can be pointed upward instead of downward. FIG. 13 is a schematic front view of the movement restriction structure 1102 that shows the differing orientations of the first body member 1104 and the second body member 1106.

It will be appreciated that the balloon can be loaded into the movement restriction structure in various ways. For example, in some embodiments, the balloon catheter can simply be threaded through the movement restriction structure before or after being connected with other portions of the apparatus in preparation for coating. In other embodiments, the movement restriction structure itself can be manipulated in order to load the balloon. For example, in some embodiments, the movement restriction structure can be rotated into an open orientation in order to accommodate loading the balloon from the side. Then, in some embodiments, the movement restriction structure can be rotated from the open orientation to a closed orientation in order to lock the balloon in place. Referring now to FIG. 14, a schematic front view of the movement restriction structure 1102 is shown illustrating an open orientation. In this view, it can be seen that the first body member 1104 and the second body member 1106 are rotated approximately 90 degrees from their respective positions in FIG. 13. The balloon 1118 can be slid out from between the first and second body members 1104, 1106 when the movement restriction structure 1102 is in this orientation. In operation, then, a new balloon to be coated can be slid back in between the first and second body members 1104, 1106 and then the body members can be rotated in the direction of arrows 1150 and 1152 to put the movement restriction structure 1102 into the closed position (illustrated in FIG. 13) where the balloon 1118 is locked in place. In some embodiments, the first and second body members 1104, 1106 can be rotated in either direction. The first and second body members 1104, 1106 can be rotated together around a single axis or independently from one another around two separate axes.

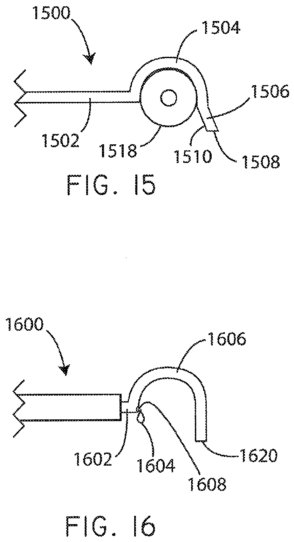

It will be appreciated that body members of movement restrictions structures in accordance with embodiments herein can also include various other features. Referring now to FIG. 15, a schematic end view of portions of a movement restriction structure 1500 are shown in accordance with various embodiments herein. The movement restriction structure 1500 can include a first body member 1502. The first body member 1502 can include a curved segment 1504 and an end 1508. The curved segment 1504 can define a portion of a channel which can surround at least a portion of the balloon 1518, thereby restricting the balloon's 1518 movement, in conjunction with a second body member (not shown in this view). The first body member 1502 can also include an alignment lip 1506 adjacent to the end 1508. The alignment lip 1506 can include a surface 1510 that is angled away from the channel defined by the curved segment 1504. The alignment lip 1506 can aid in positioning the balloon 1518 within the channel formed by the curved segment 1504. For example, when the first body member 1502 is rotated starting from the open position, if the balloon 1518 is slightly out of position by being too close to the end 1508, the surface 1510 of the alignment lip 1506 will contact the balloon 1518 surface and cause the balloon 1518 to move into alignment with the channel.

It will be appreciated that fluid applicators can take on various configurations in accordance with embodiments herein. FIG. 16 is a schematic end view of a fluid applicator 1600 in accordance with various embodiments herein. The fluid applicator 1600 can include a shaft 1602 and an orifice 1608. The orifice 1608 can be located along the shaft 1602 at a position other than at the distal end 1620 of the shaft 1602. Fluid 1604, such as a coating solution, can pass from the fluid applicator 1600 through the orifice 1608 in order to be deposited on the surface of the balloon. The segment 1606 of the shaft 1602 that extends beyond where the orifice 1608 is located can be curved, in some embodiments, in order to form part of a channel which can serve to maintain the position of the balloon relative to the fluid applicator 1600. In some embodiments, segment 1606 can be disposed between the orifice 1608 and the distal end 1620 of the shaft 1602.

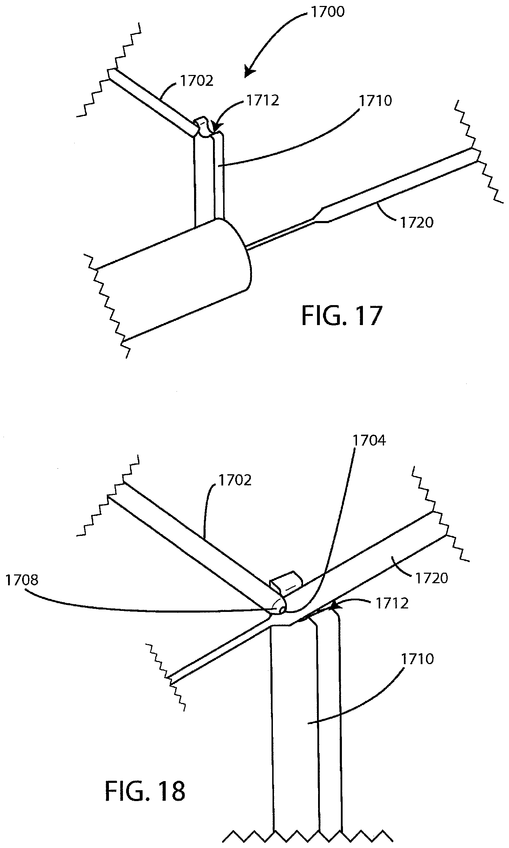

Referring now to FIG. 17, a schematic perspective view of a coating apparatus 1700 is shown in accordance with various embodiments herein. The coating apparatus 1700 can include a fluid applicator 1702 and a movement restriction structure 1710. The movement restriction structure 1710 can include an engagement surface 1712 having a U-shaped contour. A device to be coated, such as a balloon catheter 1720, is shown as ready for insertion into the movement restriction structure 1710.

Referring now to FIG. 18, a schematic perspective view is shown of a fluid applicator 1702 interfacing with balloon catheter 1720 as held by a movement restriction structure 1710. The movement restriction structure 1710 can include an engagement surface 1712 having a U-shaped contour. The fluid applicator 1702 can include a contact surface 1708 that is angled with respect to the lengthwise axis of the fluid applicator 1702. The contact surface 1708 can fit against the balloon catheter during the coating operation so as to make contact and aid in holding the balloon catheter against the engagement surface 1712 of the movement restriction structure. 19 is a schematic perspective view of a fluid applicator 1702 interfacing with balloon catheter 1720 as held by a movement restriction structure 1710. A fluid coating composition 1722 is applied onto the surface of the balloon catheter 1720. The fluid coating composition 1722 then passes past a drying nozzle 1726 forming a dried coating 1724.

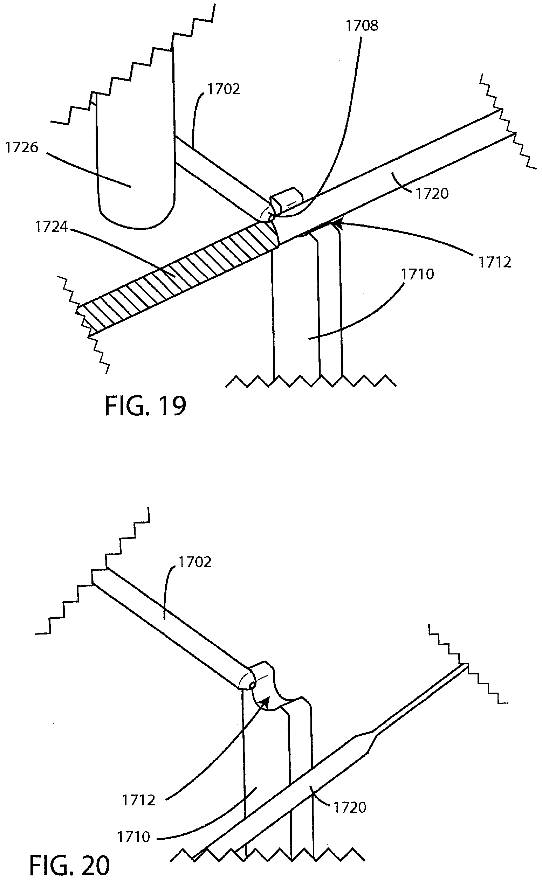

Referring now to FIG. 20, a schematic perspective view of elements of a coating apparatus in accordance with various embodiments herein. In this view, a coating operation has been completed and the now coated balloon catheter 1720 has been removed from contact with the fluid applicator 1702 and has been moved out from within the engagement surface 1712 of the movement restriction structure 1710.

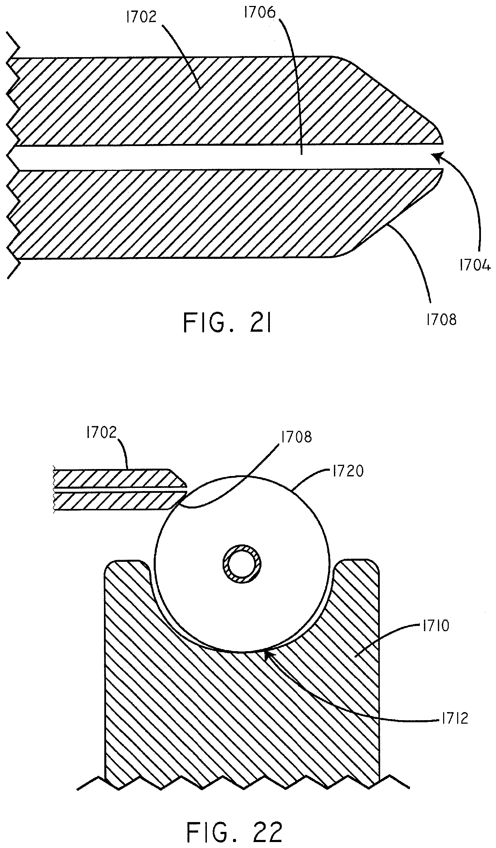

Referring now to FIG. 21, a schematic cross-sectional view through the middle of a fluid applicator 1702 is shown in accordance with various embodiments herein. The fluid applicator 1702 can be formed of various materials including polymers, metals, ceramics, glasses, and the like. The fluid applicator 1702 can define a central channel 1706 through which a fluid coating composition can be delivered. The fluid applicator 1702 can further include a contact surface 1708. The contact surface 1708 can be angled with respect to the lengthwise axis of the fluid applicator 1702. In some embodiments, the contact surface 1708 can be angled from about 20 degrees to about 70 degrees with respect to the lengthwise axis of the fluid applicator 1702. In some embodiments, the contact surface 1708 is angled such that its surface is substantially parallel with a line that is a tangent to the balloon catheter 1720 at the point of contact between the fluid applicator 1702 and the balloon catheter 1720. A fluid orifice 1704 can be disposed as the distal end of the fluid applicator 1702.

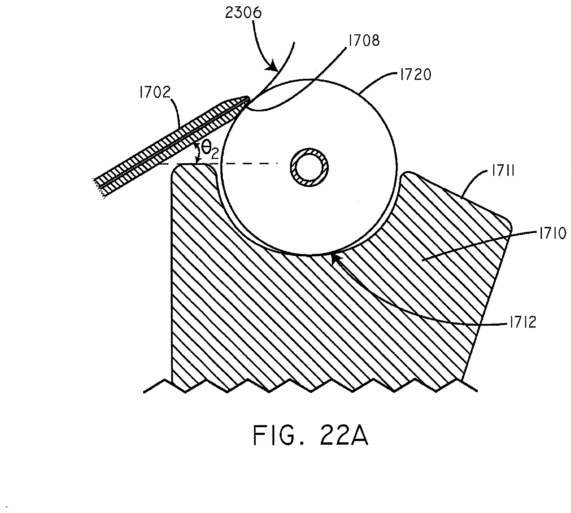

FIG. 22 is a schematic cross-sectional view of a fluid applicator 1702 interfacing with a balloon catheter 1720 as held by a movement restriction structure 1710. The contact surface 1708 of the fluid applicator 1702 can contact the surface of the balloon catheter 1720. In some embodiments, the contact point between the fluid applicator 1702 and the surface of the balloon catheter 1720 can be at a position equivalent to between 9 and 12 on the face of a standard clock (or between 12 and 3 if the fluid applicator 1702 is arranged on the other side of the balloon catheter). If the position of 3 on a clock face is taken as 0 degrees, then the contact point can be between 180 degrees and 270 degrees (or between 270 degrees and 360 degrees if the fluid applicator 1702 is arranged on the other side of the balloon catheter). The surface of the balloon catheter 1720 can contact the engagement surface 1712 of the movement restriction structure 1710. In some embodiments, the leading and trailing edges of the engagement surface 1712 can include curved edges. In some embodiments, the engagement surface 1712 can include a radius of curvature extending from the leading edge to the trailing edge such that the point of contact with the surface of the balloon catheter is in the middle of the engagement surface between the leading edge and the trailing edge.

FIG. 22A is a schematic cross-sectional view of a fluid applicator 1702 interfacing with a balloon catheter 1720 as held by a movement restriction structure 1710. The contact surface 1708 of the fluid applicator 1702 can contact the surface of the balloon catheter 1720. In some embodiments, the contact point between the fluid applicator 1702 and the surface of the balloon catheter 1720 can be at a position equivalent to between 9 and 12 on the face of a standard clock (or between 12 and 3 if the fluid applicator 1702 is arranged on the other side of the balloon catheter). If the position of 3 on a clock face is taken as 0 degrees, then the contact point can be between 180 degrees and 270 degrees (or between 270 degrees and 360 degrees if the fluid applicator 1702 is arranged on the other side of the balloon catheter). Additionally, the fluid applicator 1702 can be held at an angle θ₂ (with respect to a fully horizontal position) from the contact points described above. The angle θ₂ can vary between 0 (as shown in FIG. 22A) and as much as 30 degrees. The surface of the balloon catheter 1720 can contact the engagement surface 1712 of the movement restriction structure 1710. In some embodiments, the leading and trailing edges (e.g., oriented such that a line connecting the leading and trailing edges would be perpendicular to the cross-section of FIG. 22A and thus go into and out of the page) of the engagement surface 1712 can include curved edges. In yet other embodiments the engagement surface can include an engagement ramp 1711 to allow the balloon catheter 1720 to more easily become engaged with the engagement surface 1712. In some embodiments, the engagement surface 1712 can include a radius of curvature extending from the leading edge to the trailing edge such that the point of contact with the surface of the balloon catheter is in the middle of the engagement surface between the leading edge and the trailing edge.

In some embodiments the fluid distribution bar 2306 can act to further restrict movement and keep the balloon catheter 1720 engaged against the engagement surface 1712. In some exemplary embodiments, wherein the angle θ₂ exceeds 15 degrees, the fluid distribution bar 2306 can further restrict movement and keep the balloon catheter 1720 engaged against the engagement surface 1712.

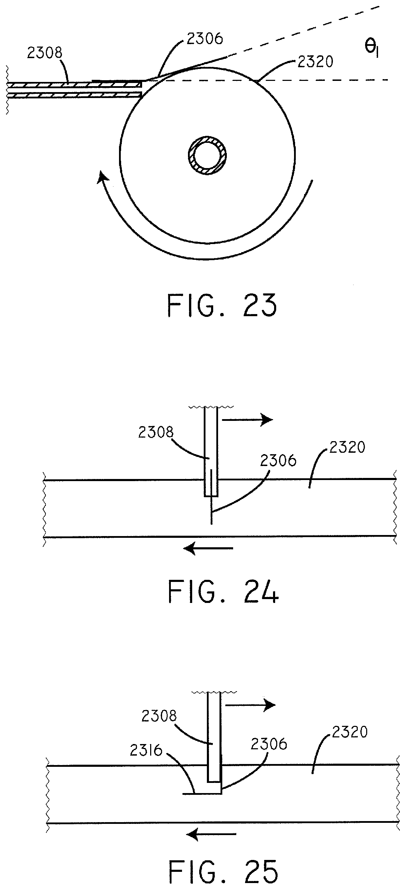

Referring now to FIG. 23, a schematic cross-sectional view is shown of a fluid applicator 2308 interfacing with the surface 2320 of a balloon catheter. The coating apparatus can further include a fluid distribution bar 2306 (or wire) that is connected to the fluid applicator 2308 and is arranged to contact the surface 2320 of the balloon catheter.

In this view, the fluid distribution bar 2306 is angled upward with respect to device to be coated such that it contacts the device to be coated at a higher point than the fluid applicator. In some embodiments, the fluid distribution bar is arranged such that it does not actually contact the surface of the device to be coated but is sufficiently close so as to contact any coating material that is deposited thereon. The angle (θ₁) of the body of the fluid distribution bar 2306 with respect to the lengthwise major axis of the fluid applicator 2308 and/or the tip of the fluid applicator 2308 can be from about 0 degrees to about 20 degrees. In some embodiments the angle (θ₁) is from about 0.5 degrees to about 20 degrees. In some embodiments, the angle is sufficient such that the body of the fluid distribution bar 2306 is tangent with respect to the surface of the device to be coated. In other embodiments, the fluid distribution bar is not be angled upward.

In this view, the fluid distribution bar 2306 is shown connected to the top side of the fluid applicator 2308. However, it will be appreciated that the fluid distribution bar 2306 can also be connected to the sides, bottom, or any other portion of the fluid applicator in other embodiments. In still other embodiments, the fluid distribution bar is not connected to the fluid applicator at all. FIG. 24 is a schematic top view of the fluid applicator 2308 and fluid distribution bar 2306 interfacing with the surface 2320 balloon catheter.

While the embodiment shown in 24 illustrates an embodiment of a fluid distribution bar 2306 that is substantially straight, it will be appreciated that in other embodiments the fluid distribution bar can be curved and/or have bent portions. 25 a schematic top view of a fluid applicator interfacing with a balloon catheter is shown in accordance with another embodiment. The coating apparatus includes a fluid distribution bar 2306 that is connected to the fluid applicator 2308 and is arranged to contact the surface 2320 of the balloon catheter. The fluid distribution bar 2306 also includes a portion 2316 (or tail) that is angled with respect to the major axis of the fluid applicator. The portion 2316 extends in a direction that is downstream of the fluid applicator (e.g. on the side of the fluid applicator where coating material has already been deposited based on how the fluid applicator and/or the device to be coated move with respect to one another). However, in other embodiments, the portion 2316 could extend upstream of the fluid applicator.

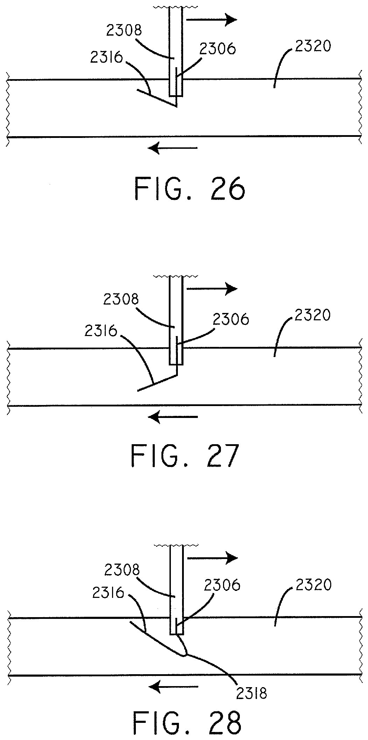

In some embodiments the portion 2316 can be substantially parallel with the lengthwise axis of the device to be coated, such as the balloon catheter. In other embodiments, the portion 2316 can be angled with respect to the lengthwise axis of the device to be coated. 26, a schematic top view is shown of a fluid applicator interfacing with a balloon catheter in accordance with various embodiments. The coating apparatus includes a fluid distribution bar 2306 that is connected to the fluid applicator 2308 and is arranged to contact the surface 2320 of the balloon catheter. The fluid distribution bar 2306 includes a portion 2316 that is angled back toward the fluid applicator 2308. FIG. 27 shows another embodiment of the coating apparatus. In this view, the fluid distribution bar 2306 includes a portion 2316 that is angled away from the fluid applicator 2308.

FIG. 28 is a schematic top view of a fluid applicator interfacing with a balloon catheter in accordance with another embodiment The coating apparatus includes a fluid distribution bar 2306 that is connected to the fluid applicator 2308 and is arranged to contact the surface 2320 of the balloon catheter. The fluid distribution bar 2306 includes a portion 2318 that is disposed upstream of the fluid applicator and a portion 2316 that is disposed downstream of the fluid applicator.

It will be appreciated that the fluid distribution bar can be made of various materials. In some embodiments it can be formed of polymers, metals, composites, ceramics and the like. In some embodiments it is flexible. In other embodiments it is substantially rigid. In some embodiments it can have a diameter that is less than the diameter of the fluid applicator. In some embodiments it is circular in cross sectional. In other embodiments it can be polygonal, ovoid, or irregular in cross-section. In some embodiments it can have a flattened surface where it contacts the device to be coated.

It will be appreciated that coating solutions applied onto balloons can include various components including, but not limited to, one or more active agents, carrier agents and/or solvents, polymers (including degradable or non-degradable polymers), excipients, and the like. The relative amounts of the components of the coating solution will depend on various factors including the desired amount of active agent to be applied to the balloon and the desired release rate of the active agent.

Embodiments herein include methods of applying coatings onto balloon catheters. In an embodiment, the method can include rotating a balloon catheter with a rotation mechanism, the balloon catheter comprising a balloon, contacting the balloon with a movement restriction structure defining a channel, wherein the channel limits lateral movement of the balloon, applying a coating solution onto the surface of the balloon with a fluid applicator (such as through direct contact with a fluid applicator), contacting the surface of the balloon with a fluid distribution bar, and blowing a stream of a gas onto the surface of the balloon. In some embodiments, the balloon catheter can be rotated at a speed of between 100 and 400 rotations per minute.

In some embodiments, the method can include moving the fluid applicator relative to the lengthwise axis of the drug eluting balloon catheter. In some embodiments, the method can include moving the drug eluting balloon catheter along its lengthwise axis relative to the fluid applicator, fluid distribution bar, and movement restriction structure.

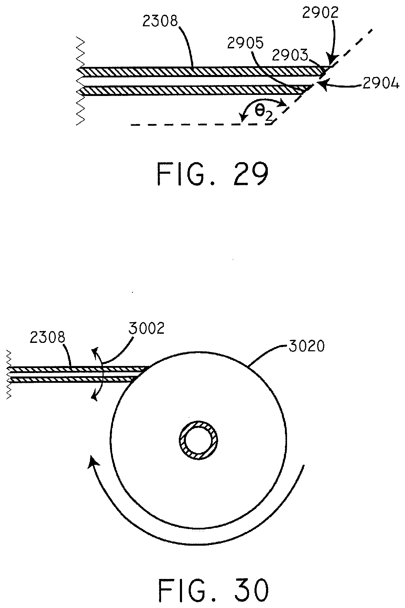

In FIG. 29 a schematic cross-sectional view is shown of a fluid applicator 2308. The fluid applicator 2308 includes a tip 2902 having a face 2904 (or surface) that is angled with respect to the lengthwise axis of the fluid applicator 2308. The face 2904 extends across the width of the tip 2902. As shown, a line that connects the top portion 2903 of the tip 2902 with the bottom portion 2905 of the tip 2902 (or the line is parallel to the face 2904 of the tip 2902) has an angle Θ2 with respect to a line parallel to the lengthwise axis of the fluid applicator 2308. Angle Θ2 can be from about 105 to about 165 degrees.

FIG. 30 shows a view of the fluid applicator 2308 interfacing with a surface 3020 of a device to be coated, such as a balloon catheter. The fluid applicator 2308 is configured to rotate around its lengthwise axis as indicated by arrow 3002, and is connected to a rotation mechanism to cause it to rotate.

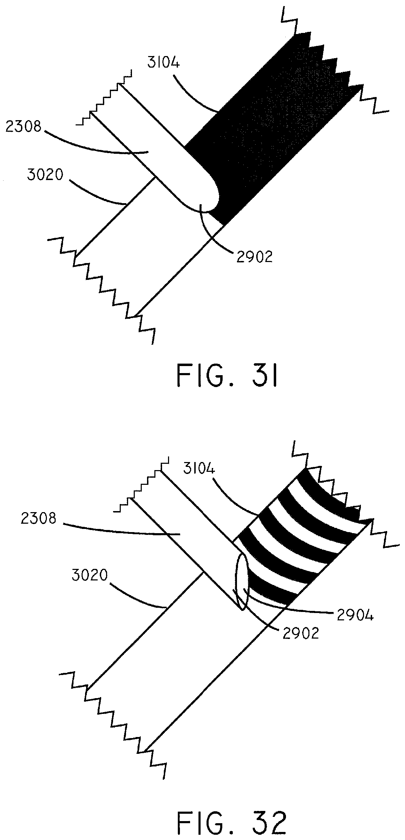

Referring now to FIG. 31, a schematic top view is shown of a fluid applicator 2308 interfacing with a surface 3020 of a device to be coated. In this view, the face of the fluid applicator 2308 is oriented downward toward the surface 3020 consistent with the orientation shown in FIG. 30. It has been found that this orientation of the tip face 2904 results in a coating 3104 being applied that is substantially continuous in coverage. In FIG. 32, a schematic top view is shown of a fluid applicator 2308 interfacing with a surface 3020 of a device to be coated wherein the face 2904 of the tip 2902 is oriented differently than as shown in FIG. 31. In this view, the fluid applicator 2308 has been rotated approximately 90 degrees such that the face 2904 of the tip 2902 is now facing approximately sideward with respect to the surface 3020. It has been found that this orientation of the tip face 2904 results in a coating 3104 being applied that is discontinuous in coverage and forms a spiral or helical shape.

The fluid applicator 2308 can be rotated from about 10 degrees to about 170 degrees.

In various embodiments, the fluid applicator 2308 can be rotated to a particular orientation in order to create either a continuous or discontinuous coating as desired. In some embodiments, the fluid applicator 2308 can be rotated during a coating application process allowing for the creation of medical devices having segments of continuous coating coverage and segments with discontinuous coating coverage.

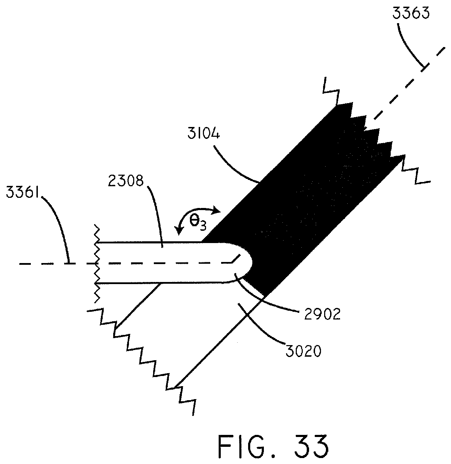

Referring now to FIG. 33, a schematic top view is shown of a fluid applicator 2308 interfacing with a surface 3020 of a device to be coated. In this view, the lengthwise axis 3361 of the fluid applicator 2308 forms an angle Θ3 with respect to the lengthwise axis 3363 of the device to be coated. In specific, angle Θ3 can be from about 0 to about 180 degrees. In some embodiments, angle Θ3 can be from about 20 to about 160 degrees. In some embodiments, angle Θ3 can be from about 45 to about 135 degrees. In some embodiments, angle Θ3 can be from about 70 to about 110 degrees. In some embodiments, angle Θ3 can be from about 85 to about 95 degrees. In some embodiments, angle Θ3 can be about 90 degrees. In some embodiments, angle Θ3 can be from about 115 degrees to about 155 degrees. In some embodiments, angle Θ3 can be from about 125 degrees to about 145 degrees.

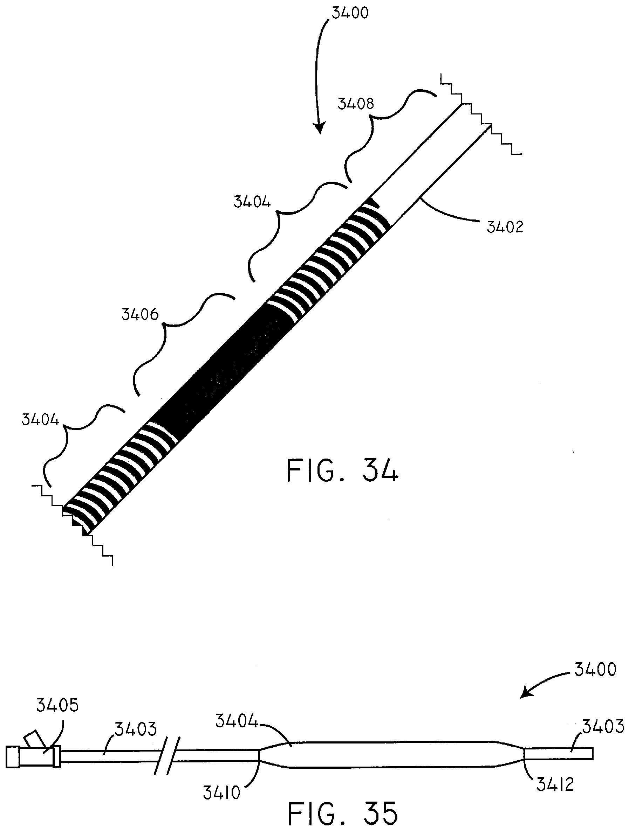

Referring now to FIG. 34, a schematic view is show of a portion of a medical device 3400 having a surface 3402. The surface 3402 includes a segment 3406 with continuous coating coverage and segments 3404 with discontinuous coating coverage. The segment with discontinuous coating coverage can have various shaped coating configurations such as spiral, helical, ring, and the like. In some embodiments, the surface 3402 can also include a segment 3408 with no coating coverage.

It will be appreciated that the segments can be aligned with various features of the device to be coated. By way of example, to decrease the amount of coating material in an area near the ends of a balloon on a balloon catheter, the fluid applicator can be rotated during the coating process to produce a discontinuous coating segment when approaching the ends of the balloon.

Referring now to FIG. 35, a schematic view of a medical device 3400 is shown. The medical device 3400 can optionally include a connection manifold 3405, a shaft 3403 having a surface, and an expandable portion 3404 (such as a balloon) having a surface. The expandable portion 3404 can include a proximal end 3410 and a distal end 3412. Coating segments, such as those shown in FIG. 34, can be disposed onto one or more of the shaft 3403 and the expandable portion 3404. In some embodiments, the expandable portion 3404 can include multiple coating segments thereon disposed adjacently to one another. By way of example, the expandable portion 3404 can include both continuous coating coverage segments and discontinuous coating coverage segments.

In some embodiments, the medical device 3400 can include at least one continuous coverage segment and at least two discontinuous coverage segments in some embodiments. In some embodiments, the discontinuous coverage segment can be disposed over at least one of the distal end and the proximal end. In some embodiments, a first discontinuous coverage segment can be disposed over the proximal end and a second discontinuous coverage segment can be disposed over the distal end. In some embodiments, the continuous coverage segment can be disposed over the expandable portion between the distal end and the proximal end.

In various embodiments, a method of coating a medical device is included. The method includes rotating a medical device to be coated with a rotation mechanism. In some embodiments, the medical device can be a balloon catheter comprising a balloon. The method further includes contacting the surface of the medical device with a fluid applicator, applying a coating solution onto the surface of the balloon with the fluid applicator, and rotating the fluid applicator axially about its lengthwise major axis.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration to. The phrase "configured" can be used interchangeably with other similar phrases such as arranged and configured, constructed and arranged, constructed, manufactured and arranged, and the like.

All publications and patent applications in this specification are indicative of the level of ordinary skill in the art to which this invention pertains.

The invention has been described with reference to various specific and preferred embodiments and techniques. However, it should be understood that many variations and modifications may be made while remaining within the scope of the claims.

## Claims

1. A coating apparatus (1700) comprising:
a coating application unit (108) comprising
a movement restriction structure (1710); and
a fluid applicator (2308) having a lengthwise axis (3361) and a width, the fluid applicator (2308) comprising a tip (2902), the tip (2902) comprising a face (2904) across the width of the fluid applicator (2308), the face (2904) oriented at an angle (θ₂) of from about 105 to about 165 degrees with respect to the lengthwise axis (3361) of the fluid applicator (2308), the fluid applicator (2308) configured to rotate (3002) around its lengthwise axis (3361);
a rotation mechanism (116); and
an axial motion mechanism (110, 118), the axial motion mechanism (110, 118) configured to cause movement of at least one of the coating application unit (108) and the rotation mechanism (116) with respect to one another
**characterised in that** the fluid applicator (2308) is configured to rotate (3002) around its lengthwise axis (3361) by an amount from about 10 degrees to about 170 degrees.

2. The coating apparatus (1700) of claim 1, wherein the fluid applicator (2308) is configured to be rotated (3002) during a coating process.

3. The coating apparatus (1700) of claim 1, wherein the lengthwise axis (3361) of the fluid applicator (2308) forms an angle (θ₃) with respect to the lengthwise axis (3363) of a device (3400) to be coated.

4. The coating apparatus (1700) of claim 3, wherein the angle (θ₃) of the lengthwise axis (3361) of the fluid applicator (2308) with respect to the lengthwise axis (3363) of the device (3400) to be coated is from about 20 to about 160 degrees.

5. The coating apparatus (1700) of claim 1, the movement restriction structure (1710) comprising an engagement surface (1712) having a U-shaped contour.

6. The coating apparatus (1700) of claim 1, wherein the rotation mechanism (116) is configured to rotate a balloon catheter (1720) around its lengthwise axis at a speed of between 100 and 400 rotations per minute.

7. A method of coating a medical device (3400) comprising:
engaging a medical device (3400) to be coated into a movement restriction structure (1710);
rotating the medical device (3400) with a rotation mechanism (116);
contacting the surface (3402) of the medical device (3400) with a fluid applicator (2308) having a lengthwise axis (3361) and a width, the fluid applicator (2308) comprising a tip (2902), the tip (2902) comprising a face (2904) across the width of the fluid applicator (2308), the face (2904) oriented at an angle (θ₂) of from about 105 to about 165 degrees with respect to the lengthwise axis (3361) of the fluid applicator (2308);
applying a coating solution (3104) onto the surface (3402) of a balloon (3404) of the medical device (3400) with the fluid applicator (2308); and **characterised by**
rotating the fluid applicator (2308) about its lengthwise axis (3361) by an amount from about 10 degrees to about 170 degrees.

8. The method of coating a medical device (3400) of claim 7, further comprising applying a coating solution (3104) onto the surface (3402) of the balloon (3404) with the fluid applicator (2308) after rotating the fluid applicator (2308) about its lengthwise axis (3361).

9. The method of coating a medical device (3400) of claim 7, further comprising rotating the medical device (3400) to be coated with the rotation mechanism (116) at a speed of between 100 and 400 rotations per minute.

10. The method of coating a medical device (3400) of claim 7, the movement restriction structure (1710) comprising an engagement surface (1712) having a U-shaped contour.

11. The method of coating a medical device (3400) of claim 7, wherein the coating (3104) applied to the medical device (3400) includes a continuous coverage segment (3406) and a discontinuous coverage segment (3404).

12. The method of coating a medical device (3400) of claim 7, wherein the coating (3104) applied to the medical device (3400) includes at least one of a polymer and an active agent.

13. The method of coating a medical device (3400) of claim 7, further comprising moving the fluid applicator (2308) with respect to the medical device (3400) to be coated with an axial motion mechanism (110, 118) while the fluid applicator (2308) contacts the surface (3402) of the medical device (3400).

14. The method of coating a medical device (3400) of claim 7, wherein the medical device (3400) is a balloon catheter.

## Patentansprüche

1. Eine Beschichtungsvorrichtung (1700), die Folgendes umfasst:
eine Beschichtungsauftragseinheit (108), die Folgendes umfasst
eine Bewegungsbegrenzungsstruktur (1710); und
einen Fluidapplikator (2308) mit einer Längsachse (3361) und einer Breite, wobei der Fluidapplikator (2308) eine Spitze (2902) umfasst, wobei die Spitze (2902) eine Fläche (2904) über die Breite des Fluidapplikators (2308) umfasst, die Fläche (2904) in einem Winkel (θ₂) von etwa 105 bis etwa 165 Grad in Bezug auf die Längsachse (3361) des Fluidapplikators (2308) ausgerichtet ist, wobei der Fluidapplikator (2308) so konfiguriert ist, dass er sich um seine Längsachse (3361) dreht (3002);
einen Drehmechanismus (116); und
einen Axialbewegungsmechanismus (110, 118), wobei der Axialbewegungsmechanismus (110, 118) so konfiguriert ist, dass er eine Bewegung der Beschichtungsauftragseinheit (108) und/oder des Drehmechanismus (116) in Bezug zueinander bewirkt **dadurch gekennzeichnet, dass** der Fluidapplikator (2308) so konfiguriert ist, dass er sich um seine Längsachse (3361) um einen Betrag von etwa 10 Grad bis etwa 170 Grad dreht (3002).

2. Die Beschichtungsvorrichtung (1700) nach Anspruch 1, wobei der Fluidapplikator (2308) so konfiguriert ist, dass er während eines Beschichtungsprozesses gedreht wird (3002) .

3. Die Beschichtungsvorrichtung (1700) nach Anspruch 1, wobei die Längsachse (3361) des Fluidapplikators (2308) einen Winkel (θ₃) in Bezug auf die Längsachse (3363) eines zu beschichtenden Geräts (3400) bildet.

4. Die Beschichtungsvorrichtung (1700) nach Anspruch 3, wobei der Winkel (θ₃) der Längsachse (3361) des Fluidapplikators (2308) in Bezug auf die Längsachse (3363) des zu beschichtenden Geräts (3400) etwa 20 bis etwa 160 Grad beträgt.

5. Die Beschichtungsvorrichtung (1700) nach Anspruch 1, wobei die Bewegungseinschränkungsstruktur (1710) eine Eingriffsfläche (1712) mit einer U-förmigen Kontur umfasst.

6. Die Beschichtungsvorrichtung (1700) nach Anspruch 1, wobei der Drehmechanismus (116) so konfiguriert ist, dass er einen Ballonkatheter (1720) um seine Längsachse mit einer Geschwindigkeit zwischen 100 und 400 Umdrehungen pro Minute dreht.

7. Ein Verfahren zum Beschichten eines medizinischen Geräts (3400), welches Folgendes umfasst:
Einsetzen eines zu beschichtenden medizinischen Geräts (3400) in eine Bewegungseinschränkungsstruktur (1710);
Drehen des medizinischen Geräts (3400) mit einem Drehmechanismus (116);
Inkontaktbringen der Oberfläche (3402) des medizinischen Geräts (3400) mit einem Fluidapplikator (2308) mit einer Längsachse (3361) und einer Breite, wobei der Fluidapplikator (2308) eine Spitze (2902) umfasst, wobei die Spitze (2902) eine Fläche (2904) über die Breite des Fluidapplikators (2308) umfasst, die Fläche (2904) in einem Winkel (θ₂) von etwa 105 bis etwa 165 Grad in Bezug auf die Längsachse (3361) des Fluidapplikators (2308) ausgerichtet ist;
Auftragen einer Beschichtungslösung (3104) auf die Oberfläche (3402) eines Ballons (3404) des medizinischen Geräts (3400) mit dem Fluidapplikator (2308); und **gekennzeichnet durch**
das Drehen des Fluidapplikators (2308) um seine Längsachse (3361) um einen Betrag von etwa 10 Grad bis etwa 170 Grad.

8. Das Verfahren zum Beschichten eines medizinischen Geräts (3400) nach Anspruch 7, ferner umfassend das Auftragen einer Beschichtungslösung (3104) auf die Oberfläche (3402) des Ballons (3404) mit dem Fluidapplikator (2308) nach dem Drehen des Fluidapplikators (2308) um seine Längsachse (3361).

9. Das Verfahren zum Beschichten eines medizinischen Geräts (3400) nach Anspruch 7, das ferner das Drehen des zu beschichtenden medizinischen Geräts (3400) mit dem Drehmechanismus (116) mit einer Geschwindigkeit zwischen 100 und 400 Umdrehungen pro Minute umfasst.

10. Das Verfahren zum Beschichten eines medizinischen Geräts (3400) nach Anspruch 7, wobei die Bewegungseinschränkungsstruktur (1710) eine Eingriffsfläche (1712) mit einer U-förmigen Kontur umfasst.

11. Das Verfahren zur Beschichtung eines medizinischen Geräts (3400) nach Anspruch 7, wobei die auf das medizinische Gerät (3400) aufgebrachte Beschichtung (3104) ein kontinuierliches Bedeckungssegment (3406) und ein diskontinuierliches Bedeckungssegment (3404) umfasst.

12. Das Verfahren zur Beschichtung eines medizinischen Geräts (3400) nach Anspruch 7, wobei die auf das medizinische Gerät (3400) aufgebrachte Beschichtung (3104) mindestens eines von einem Polymer und einem Wirkstoff enthält.

13. Das Verfahren zum Beschichten eines medizinischen Geräts (3400) nach Anspruch 7, das ferner das Bewegen des Fluidapplikators (2308) in Bezug auf das zu beschichtende medizinische Gerät (3400) mit einem axialen Bewegungsmechanismus (110, 118) umfasst, während der Fluidapplikator (2308) die Oberfläche (3402) des medizinischen Geräts (3400) berührt.

14. Das Verfahren zum Beschichten eines medizinischen Geräts (3400) nach Anspruch 7, wobei das medizinische Gerät (3400) ein Ballonkatheter ist.

## Revendications

1. Appareil de revêtement (1700) comprenant :
une unité d'application de revêtement (108) comprenant
une structure de restriction de mouvement (1710) ; et
un applicateur de fluide (2308) ayant un axe longitudinal (3361) et une largeur, l'applicateur de fluide (2308) comprenant un embout (2902), l'embout (2902) comprenant une face (2904) sur la largeur de l'applicateur de fluide (2308), la face (2904) étant orientée à un angle (θ₂) d'environ 105 à environ 165 degrés par rapport à l'axe longitudinal (3361) de l'applicateur de fluide (2308), l'applicateur de fluide (2308) étant configuré pour tourner (3002) autour de son axe longitudinal (3361) ;
un mécanisme de rotation (116) ; et
un mécanisme de mouvement axial (110, 118), le mécanisme de mouvement axial (110, 118) étant configuré pour provoquer le mouvement d'au moins une parmi l'unité d'application de revêtement (108) et le mécanisme de rotation (116) l'un par rapport à l'autre
**caractérisé en ce que** l'applicateur de fluide (2308) est conçu pour tourner (3002) autour de son axe longitudinal (3361) d'une valeur d'environ 10 degrés à environ 170 degrés.

2. Appareil de revêtement (1700) selon la revendication 1, dans lequel l'applicateur de fluide (2308) est conçu pour être tourné (3002) pendant un processus de revêtement.

3. Appareil de revêtement (1700) selon la revendication 1, dans lequel l'axe longitudinal (3361) de l'applicateur de fluide (2308) forme un angle (θ₃) par rapport à l'axe longitudinal (3363) d'un dispositif (3400) à revêtir.

4. Appareil de revêtement (1700) selon la revendication 3, dans lequel l'angle (θ₃) de l'axe longitudinal (3361) de l'applicateur de fluide (2308) par rapport à l'axe longitudinal (3363) du dispositif (3400) à revêtir est d'environ 20 à environ 160 degrés.

5. Appareil de revêtement (1700) selon la revendication 1, la structure de restriction de mouvement (1710) comprenant une surface de mise en prise (1712) ayant un contour en forme de U.

6. Appareil de revêtement (1700) selon la revendication 1, dans lequel le mécanisme de rotation (116) est conçu pour faire tourner un cathéter à ballonnet (1720) autour de son axe longitudinal à une vitesse comprise entre 100 et 400 rotations par minute.

7. Procédé de revêtement d'un dispositif médical (3400) comprenant :
la mise en prise d'un dispositif médical (3400) à revêtir dans une structure de restriction de mouvement (1710) ;
la rotation du dispositif médical (3400) avec un mécanisme de rotation (116) ;
la mise en contact de la surface (3402) du dispositif médical (3400) avec un applicateur de fluide (2308) ayant un axe longitudinal (3361) et une largeur, l'applicateur de fluide (2308) comprenant une pointe (2902), la pointe (2902) comprenant une face (2904) sur la largeur de l'applicateur de fluide (2308), la face (2904) étant orientée à un angle (θ₂) d'environ 105 à environ 165 degrés par rapport à l'axe longitudinal (3361) de l'applicateur de fluide (2308) ;
l'application d'une solution de revêtement (3104) sur la surface (3402) d'un ballonnet (3404) du dispositif médical (3400) avec l'applicateur de fluide (2308) ; et
**caractérisé par**
la rotation de l'applicateur de fluide (2308) autour de son axe longitudinal (3361) d'une valeur comprise entre environ 10 degrés et environ 170 degrés.

8. Procédé de revêtement d'un dispositif médical (3400) selon la revendication 7, comprenant en outre l'application d'une solution de revêtement (3104) sur la surface (3402) du ballonnet (3404) avec l'applicateur de fluide (2308) après la rotation de l'applicateur de fluide (2308) autour de son axe longitudinal (3361).

9. Procédé de revêtement d'un dispositif médical (3400) selon la revendication 7, comprenant en outre la rotation du dispositif médical (3400) à revêtir avec le mécanisme de rotation (116) à une vitesse comprise entre 100 et 400 rotations par minute.

10. Procédé de revêtement d'un dispositif médical (3400) selon la revendication 7, la structure de restriction du mouvement (1710) comprenant une surface de mise en prise (1712) ayant un contour en forme de U.

11. Procédé de revêtement d'un dispositif médical (3400) selon la revendication 7, dans lequel le revêtement (3104) appliqué au dispositif médical (3400) comporte un segment de couverture continue (3406) et un segment de couverture discontinue (3404).

12. Procédé de revêtement d'un dispositif médical (3400) selon la revendication 7, dans lequel le revêtement (3104) appliqué au dispositif médical (3400) comporte au moins un polymère et un agent actif.

13. Procédé de revêtement d'un dispositif médical (3400) selon la revendication 7, comprenant en outre le déplacement de l'applicateur de fluide (2308) par rapport au dispositif médical (3400) à revêtir avec un mécanisme de mouvement axial (110, 118) pendant que l'applicateur de fluide (2308) entre en contact avec la surface (3402) du dispositif médical (3400).

14. Procédé de revêtement d'un dispositif médical (3400) selon la revendication 7, dans lequel le dispositif médical (3400) est un cathéter à ballonnet.
